# EUROPEAN PATENT APPLICATION

(11) **EP 4 729 525 A1**
(43) Date of publication of application: **22.04.2026**
(21) Application number: 24207621.4
(22) Date of filing: 18.10.2024
(51) Int. Cl.: C07F 5/02, A61P 11/00, A61K 41/00, A61P 15/00, A61P 19/00, A61P 35/00

(54) **NEWLY SYNTHESIZED BORON-NITROGEN HETEROCYCLES AND THEIR CONJUGATES WITH PHARMACEUTICALLY UTILIZABLE SUBSTANCES**

(71) Applicant: Julius-Maximilians-Universität Würzburg, 97070 Würzburg (DE)
(72) Inventor: Braunschweig, Holger, 97078 Würzburg (DE); Meinel, Lorenz, 97082 Würzburg (DE); Wüst, Leonie, 97072 Würzburg (DE); Keim, Timo, 97074 Würzburg (DE); Steinlein, Oskar, 97072 Würzburg (DE)
(74) Representative: Engelhard, Markus

(57) **Abstract**

The present invention relates to H₂O₂-sensitive compounds comprising a boron-nitrogen-heterocycle and their therapeutic applications. Moreover, the present invention relates to H₂O₂-sensitive compounds comprising a boron-nitrogen heterocycle for the targeted and controllable release of an active substance/dye/fluorophore in tissues. The present invention further relates to H₂O₂-sensitive compounds comprising a boron-nitrogen heterocycle that maybe coupled to said substance/dye/fluorophore, optionally via a linker, preferably via a selfimmolative linker. The present invention further relates to a kit of pharmaceutical ingredients that comprises said compound and a peptide or a protein that enhances the release, distribution and/or the half-life of the active substance/dye/fluorophore in target tissues. Moreover, the present invention relates to a method for the diagnosis, prevention and/or treatment of a disease, such as a proliferative disease, a neurodegenerative disease, a viral infection, and/or a non-viral infection, comprising the administration of said compound or said kit to a subject in need thereof.

## Description

### FIELD OF THE INVENTION

The present invention relates to the general field of drug delivery systems. In particular, the present invention relates to H₂O₂-sensitive compounds comprising a boron-nitrogen heterocycle and their therapeutic applications. Moreover, the present invention relates to H₂O₂-sensitive compounds comprising a boron-nitrogen heterocycle for the targeted and controllable release of an active substance/dye/fluorophore in tissues.

### BACKGROUND OF THE INVENTION

A major problem in the effective treatment of diseases is the undesirable side effects caused by the non-targeted effectiveness of the active substance used. For example, commercially used chemotherapeutic agents not only attack diseased cells but also healthy cells. Drug delivery systems (DDS) are frequently used in the pharmaceutical industry to minimize the side effects of active ingredients as much as possible. By activating these systems in the diseased tissue, the targeted release and mode of action of the previously bound and thus (partially) inactive active ingredients is made possible (activity on demand concept, AoD). Thus, these systems are intended to keep the concentration of active substances used in diseased tissue as constant as possible over a longer period of time, whereas the concentration of active substances in healthy tissue should be as low as possible.

In 1952, the first formulation that specifically released active ingredients was developed. Based on this, oral and transdermal drug delivery systems were developed in the first generation of drug delivery. Mechanisms for targeted drug release were also investigated. Second-generation drug delivery systems are based on long-term depot formulations and nanotechnology-based systems, among other things. The release or activation is triggered by pathophysiological differences such as pH, temperature, light, proteases and ROS between healthy and diseased tissue.

However, despite significant advancements in the field of targeted drug delivery, there remains a critical need for further improvement in targeted drug release systems. Current methods, while promising, often face challenges such as limited specificity, suboptimal drug release kinetics, and potential off-target effects. These limitations can result in reduced therapeutic efficacy and increased side effects, undermining the need for improved of targeted therapies. Additionally, the complexity of biological environments and the heterogeneity of diseases, particularly cancer, necessitate the development of more sophisticated and adaptable drug delivery systems. By enhancing the precision, efficiency, and safety of targeted drug release, it would be possible to improve patient outcomes, minimize adverse effects, and expand the applicability of these therapies to a broader range of conditions.

To this day, the development of drug delivery systems is a key area of research in the pharmaceutical industry, as in addition to the expected minimization of undesirable side effects, the drug dose can also be significantly reduced in many cases.

Therefore, there is a need for compounds or pharmaceutically acceptable salts thereof that enable the targeted and controllable release of an active substance/dye/fluorophore in tissues. Also, there is a need for compounds that enable the finetuning of said targeted and controllable release of the active substance/dye/fluorophore in (diseased) tissues. Moreover, there is a need for a kit of pharmaceutical ingredients that comprises said compound and a peptide or a protein that enhances the release, distribution and/or the half-life of the active substance/dye/fluorophore in target tissues. Furthermore, there is a need for a method for the diagnosis, prevention and/or treatment of a disease, such as a proliferative disease, a neurodegenerative disease, a viral infection, and/or a non-viral infection, comprising the administration of said compound or said kit to a subject in need thereof.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a compound having a general formula of any one of formulas (I) to (VII) 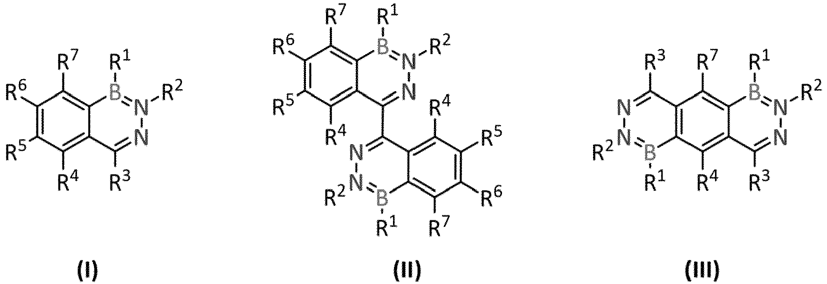 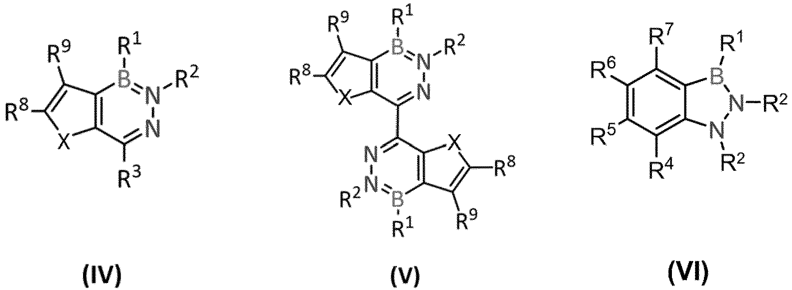 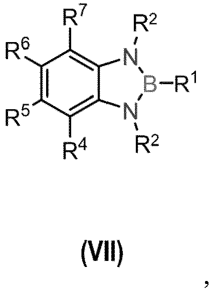 wherein
**R¹** is or comprises, at each occurrence, independently a drug or a reporter, or **R¹** is, at each occurrence, independently selected from a group comprising N(**R¹⁵**)₂, C₁-C₅ alkyl, C₁-C₂₀ alkoxycarbonyl, alkyne, aryl, alkene, and heteroaryl, wherein at least one **R¹** is or comprises a drug or a reporter,
optionally wherein the drug or the reporter is coupled and/or is attached to at least one boron atom of the compound via a linker, wherein the linker is preferably a self-immolative linker, such as an alkyl-based linker or an aryl-based linker, and/or a linker comprising an ether and/or an ester group,
**R²** to **R⁹** are, at each occurrence, independently selected from a group comprising H, OH, O**R¹⁵**, N(**R¹⁵**)₂, NO₂, SO₂, P**(R¹⁶)**₃₋₅, halogen, C₁-C₅ alkyl, C₁-C₂₀ alkoxycarbonyl, alkyne, aryl, alkene, and heteroaryl, wherein said aryl or said heteroaryl is optionally substituted with one or more of **R¹⁸,** preferably one or two of **R¹⁸,**
**X** is O, N**R¹⁵**, S, SO, or SO₂,
**R¹⁵** is, at each occurrence, independently selected from a group comprising H, OH, halogen, C₁-C₁₀ alkyl, aryl, heteroaryl, and (C=O)**R¹⁹**,
**R¹⁶** is, at each occurrence, independently O or O**R¹⁷**,
**R¹⁷** is, at each occurrence, independently selected from a group comprising P, C₁-C₁₀ alkyl, C₁-C₁₀ aryl, C₁-C₁₀ heteroaryl, and ribose,
**R¹⁸** is, at each occurrence, independently H or C₁-C₅ alkyl, and
**R¹⁹** is, at each occurrence, independently C₃-C₁₀ alkyl, optionally substituted with a maleimide group,
or a pharmaceutically acceptable salt thereof.

In one embodiment, the compound has a general formula of formula (Va)

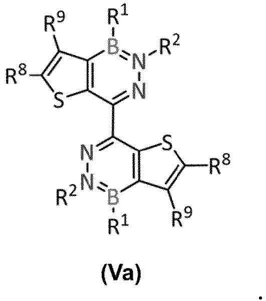

In one embodiment, at least one boron atom or nitrogen atom of the compound is chemically modified.

In one preferred embodiment, at least one boron atom of the compound is chemically modified.

In one embodiment, the linker, preferably the self-immolative linker, has a general formula of any one of formulas (A) to (F): 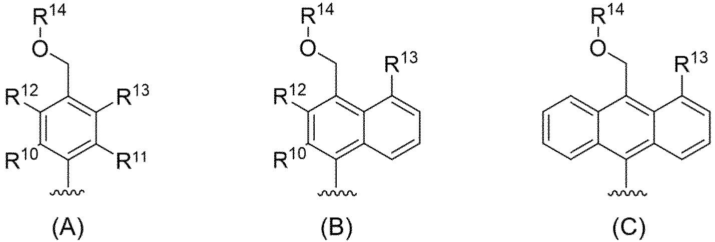 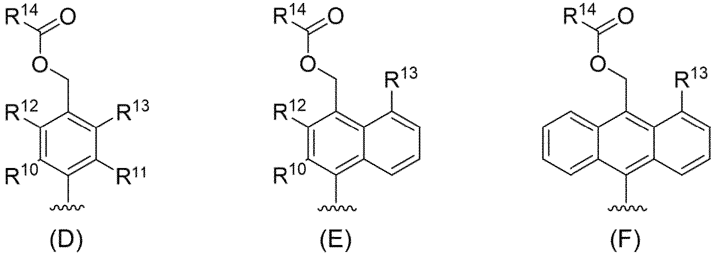 wherein **R¹⁰, R¹¹, R¹²,** and **R¹³** are each independently selected from a group comprising H, OH, O**R¹⁵**, halogen, C₁-C₁₀ alkyl, aryl, and heteroaryl, and wherein **R¹⁴** is or comprises a drug or a reporter.

In one preferred embodiment, the compound does not comprise a boronic acid function and/or borin group (B-OH) at either of the boron atoms of the heterocycle.

In one embodiment, the compound comprises one or more further moieties,
wherein the one or more further moiety/moieties is/are preferably a polymer, more preferably polyethylene glycol (PEG),
wherein said polymer is preferably attached to the compound having a general formula of any one of formulas (I) to (VII) at any of **R²** to **R⁹**, more preferably at any of **R³** to **R⁹**,
   and/or
wherein the one or more further moiety/moieties is/are preferably a peptide or a protein, such as a peptide or a protein which increases the level of reactive oxygen species (ROS), for example an antibody or an enzyme which increases the level of ROS, preferably an L-amino acid oxidase, such as Aplysia punctata ink toxin (APIT), which comprises or consists of an amino acid sequence which is at least 90% identical, preferably at least 95% identical, more preferably at least 98% identical, even more preferably at least 99% identical, optionally identical, to the amino acid sequence of SEQ ID NO. 1,
wherein said peptide or protein is preferably covalently attached to the compound having a general formula of any one of formulas (I) to (VII) at any of **R²** to **R⁹**, more preferably at any of**R³** to **R⁹**.

In one embodiment, the compound comprises one or more further moieties,
wherein the one or more further moiety/moieties is/are preferably a polymer, more preferably polyethylene glycol (PEG),
wherein said polymer is preferably attached to the compound having a general formula of any one of formulas (I) to (VII) at any of **R³** to **R⁹**,
   and/or
wherein the one or more further moiety/moieties is/are preferably a peptide or a protein, such as a peptide or a protein which increases the level of reactive oxygen species (ROS), for example an antibody or an enzyme which increases the level of ROS, preferably an L-amino acid oxidase, such as Aplysia punctata ink toxin (APIT), which comprises or consists of an amino acid sequence which is at least 90% identical, preferably at least 95% identical, more preferably at least 98% identical, even more preferably at least 99% identical, optionally identical, to the amino acid sequence of SEQ ID NO. 1,
wherein said peptide or protein is preferably covalently attached to the compound having a general formula of any one of formulas (I) to (VII) at any of **R³** to **R⁹.**

In one embodiment, if the compound comprises more than one further moiety, such as at least two further moieties, the further moieties may either be identical or the moieties may be different from each other.

In one embodiment, **R¹** or **R¹⁴** is or comprises a drug which is selected from a group comprising
anti-cancer drugs, such as antimetabolites (e.g., methotrexate), alkylating drugs, such as cylcophosphamid, topoisomerase blockers, such as etoposide, enzymes, such as asparaginase, hormonal antagonists, such as tamoxifen, mitose blockers, such as vinca alkaloids, camptothecin, anthracyclines, such as doxorubicin, kinase inhibitors, such as erlotinib, gefitinib, imatinib, or lapatinib, hormones, such as estrogens or antiestrogens (e.g., estramustin), fulvestrant, gemcitabine, cytarabine, or proteasome inhibitors, such as bortezomib,
virustatics, such as nucleosides (e.g., emitricitabin), nucleotides, such as tenofovir, or non-nucleosides, such as nevirapine, ribavirin, ganciclovir, vidarabine, acyclovir, penciclovir, zalcitabine, protease inhibitors, such as lopinavir, blockers of the integrase, such as raltegravid, entry inhibitors, such as enfuvirtide, or amantadine, and antibiotics, such as betalactam antibiotics, penicillins, aminopenicillins, acylaminopenicillins, cephalosporins, cabapenems, monobactams, aminoglycosides, streptomycin, kanamycins, tetracyclines, macrolides and their analoga, streptogramines, oxazolidones, such as linezolid, benzochinon ansamycins, such as geldanamycin, chloramphenicol, fusidic acid, mupirocin or retapamulin, gyrase inhibitors, such as fluorochinolones, folic acid antagonists, such as sulfonamides, fidaxomicin, cyclic lipopeptids, such as daptomycin, polypeptides, such as polymyxin or colistin, and antitubercular medications, such as isoniazid, pyrazinamide, or ethambutol, RNA polymerase inhibitors, such as rifampicin, or clofazimin drugs, and
antimycotic drugs, such as azol-antimycotics (e.g. voriconazole or posaconazole), polyen-antimycotics, such as amphotericin B, nystatin, or natamycin, echinocandines, such as caspofungin, anidulafungin, or micafungin.

In one embodiment, **R¹** or **R¹⁴** is or comprises a reporter which is selected from a group comprising
fluorescence dyes, such as near infrared (NIR) dyes, e.g. resorufin or 2-[2-(2,3-Dihydro-6-hydroxy-1*H*-xanthen-3-yl)ethenyl]-1-ethyl-3,3-dimethyl-3*H*-indolium,
diagnostic macromolecules,
mass reporters, and
chemoluminescence dyes.

In one embodiment, the coupling and/or attaching of the drug or the reporter to a boron-nitrogen heterocycle backbone of a compound having a general formula of any one of formulas (I) to (VII) inactivates the drug or the reporter, such as partially and/or temporarily inactivates the drug or the reporter, optionally wherein an active drug and/or an active reporter is released in presence of a reactive oxygen species (ROS), such as hydrogen peroxide (H₂O₂).

In one embodiment, the release of an active drug and/or an active reporter is directly dependent on the sensitivity of the compound to ROS, such as to H₂O₂, wherein a higher sensitivity of the compound to ROS, such as to H₂O₂, results in a faster release of an active drug and/or an active reporter and/or a lower sensitivity of the compound to ROS, such as to H₂O₂, results in a slower release of an active drug and/or an active reporter.

In one embodiment, the sensitivity of the compound to ROS, such as to H₂O₂, is dependent on steric and/or electronic characteristics of the linker, wherein the linker is preferably a self-immolative linker,
wherein the release of an active drug and/or an active reporter in presence of ROS, such as H₂O₂, is faster when the linker is (a) small and/or sterically less demanding compared to the release of an active drug and/or an active reporter in presence of ROS, such as H₂O₂, when the linker is (b) large and/or sterically demanding,
and/or wherein the release of an active drug and/or an active reporter in presence of ROS, such as H₂O₂, is slower when the linker is (a) large and/or sterically demanding compared to the release of an active drug and/or an active reporter in presence of ROS, such as H₂O₂, when the linker is (b) small and/or sterically less demanding.

In one embodiment, the sensitivity of the compound to ROS, such as to H₂O₂, is dependent on **R²,** such as is dependent on steric and/or electronic characteristics of**R²,**
wherein the release of an active drug and/or an active reporter in presence of ROS, such as H₂O₂, is faster when **R²** is (i) small and/or sterically less demanding compared to the release of an active drug and/or an active reporter in presence of ROS, such as H₂O₂, when **R²** is (ii) large and/or sterically demanding,
and/or wherein the release of an active drug and/or an active reporter in presence of ROS, such as H₂O₂, is slower when **R²** is (ii) large and/or sterically demanding compared to the release of an active drug and/or an active reporter in presence of ROS, such as H₂O₂, when **R²** is (i) small and/or sterically less demanding,
optionally wherein **R²** is (i) small and/or sterically less demanding if **R²** is H, OH, O**R¹⁵**, N(**R¹⁵**)₂, NO₂, SO₂, P**(R¹⁶)**₃₋₅, halogen, C₁-C₅ alkyl, C₁-C₅ alkoxycarbonyl, alkyne, or alkene,
   **R¹⁵** is H, OH, halogen, and
   **R¹⁶** is O,
further optionally wherein **R²** is (ii) large and/or sterically demanding if **R²** is O**R¹⁵**, N(**R¹⁵**)₂, P**(R¹⁶)**₃₋₅, C₆-C₂₀ alkoxycarbonyl, aryl, or heteroaryl, wherein said aryl or said heteroaryl is optionally substituted with one or more of **R¹⁸,** preferably one or two of**R¹⁸,**
   **R¹⁵** is C₁-C₁₀ alkyl, aryl, or heteroaryl,
   **R¹⁶** is O**R¹⁷**,
   **R¹⁷** is P, C₁-C₁₀ alkyl, C₁-C₁₀ aryl, C₁-C₁₀ heteroaryl, or ribose, and
   **R¹⁸** is, at each occurrence, independently selected from H or C₁-C₅ alkyl,
   such as wherein **R₂** is (ii) large and/or sterically demanding if **R²** is phenyl, 2-methylphenyl, 2,6-dimethylphenyl, or 2,6-diisopropylphenyl.

In one embodiment, at least one of **R²⁻⁹** is 6-Maleimidohexanamide.

In one embodiment, **R⁵** is 6-Maleimidohexanamide.

In one embodiment, the sensitivity of the compound to ROS, such as to H₂O₂, can be tuned by exchange and/or modification of the linker, preferably by changing the steric and/or electronic characteristics of the linker,
wherein the release of an active drug and/or an active reporter in presence of ROS, such as H₂O₂, is faster when the linker is (a) small and/or sterically less demanding compared to the release of an active drug and/or an active reporter in presence of ROS, such as H₂O₂, when the linker is (b) large and/or sterically demanding,
and/or wherein the release of an active drug and/or an active reporter in presence of ROS, such as H₂O₂, is slower when the linker is (a) large and/or sterically demanding compared to the release of an active drug and/or an active reporter in presence of ROS, such as H₂O₂, when the linker is (b) small and/or sterically less demanding.

In one embodiment, the sensitivity of the compound to ROS, such as to H₂O₂, can be tuned by exchange and/or modification of **R²,** preferably by changing the steric and/or electronic characteristics of **R²**,
wherein the release of an active drug and/or an active reporter in presence of ROS, such as H₂O₂, is faster when **R²** is (i) small and/or sterically less demanding compared to the release of an active drug and/or an active reporter in presence of ROS, such as H₂O₂, when **R²** is (ii) large and/or sterically demanding,
and/or wherein the release of an active drug and/or an active reporter in presence of ROS, such as H₂O₂, is slower when **R²** is (ii) large and/or sterically demanding compared to the release of an active drug and/or an active reporter in presence of ROS, such as H₂O₂, when **R²** is (i) small and/or sterically less demanding,
optionally wherein **R²** is (i) small and/or sterically less demanding if **R²** is H, OH, O**R¹⁵**, N(**R¹⁵**)₂, NO₂, SO₂, P**(R¹⁶)**₃₋₅, halogen, C₁-C₅ alkyl, C₁-C₅ alkoxycarbonyl, alkyne, or alkene,
   **R¹⁵** is H, OH, halogen, and
   **R¹⁶** is O,
further optionally wherein **R²** is (ii) large and/or sterically demanding if **R²** is O**R¹⁵**, N(**R¹⁵**)₂, P**(R¹⁶)**₃₋₅, C₆-C₂₀ alkoxycarbonyl, aryl, or heteroaryl, wherein said aryl or said heteroaryl is optionally substituted with one or more of **R¹⁸**, preferably one or two of**R¹⁸**,
   **R¹⁵** is C₁-C₁₀ alkyl, aryl, or heteroaryl,
   **R¹⁶** is O**R¹⁷**,
   **R¹⁷** is P, C₁-C₁₀ alkyl, C₁-C₁₀ aryl, C₁-C₁₀ heteroaryl, or ribose, and
   **R¹⁸** is, at each occurrence, independently selected from H or C₁-C₅ alkyl,
   such as wherein **R²** is (ii) large and/or sterically demanding if **R²** is phenyl, 2-methylphenyl, 2,6-dimethylphenyl or 2,6-diisopropylphenyl.

In one preferred embodiment, the compound is a functionalized compound.

In one embodiment, the sensitivity of the compound to ROS, such as to H₂O₂, can be tuned by adjusting the ring size and/or the placement of the heteroatoms in the boron-nitrogen heterocycle of the compound;
wherein the release of an active drug and/or an active reporter in presence of ROS, such as H₂O₂, is faster when the placement of the heteroatoms in the boron-nitrogen heterocycle is in an BNN configuration (i.e. when one nitrogen atom is placed between a boron atom and a nitrogen atom in the heterocycle, such as in compounds of any of the general formula (I) to (VI)) compared to the release of an active drug and/or an active reporter in presence of ROS, such as H₂O₂, when the placement of the heteroatoms in the boron-nitrogen heterocycle is in an NBN configuration (i.e. when one boron atom is placed between two nitrogen atoms in the heterocycle, such as in compounds of any of the general formula (VII)); and/or
wherein the release of an active drug and/or an active reporter in presence of ROS, such as H₂O₂, is slower when the placement of the heteroatoms in the boron-nitrogen heterocycle is in an NBN configuration compared to the release of an active drug and/or an active reporter in presence of ROS, such as H₂O₂, when the placement of the heteroatoms in the boron-nitrogen heterocycle is in an BNN configuration.

In one aspect, the present invention relates to a kit of pharmaceutical ingredients comprising
(1) a peptide or a protein which increases the level of reactive oxygen species (ROS), for example an antibody or an enzyme, preferably an L-amino acid oxidase, such as Aplysia punctata ink toxin (APIT), which preferably comprises or consists of an amino acid sequence which is at least 90% identical, preferably at least 95% identical, more preferably at least 98% identical, even more preferably at least 99% identical, optionally identical, to the amino acid sequence of SEQ ID NO. 1, and
(2) a compound according to any one of the foregoing aspects and embodiments.

In one aspect, the present invention relates to the use of a compound according to the present invention, or a kit according to the present invention, as a drug delivery system (DDS), in particular a DDS that releases an (active) drug and/or an (active) reporter in presence of reactive oxygen species (ROS), such as hydrogen peroxide (H₂O₂).

In one aspect, the present invention relates to a compound according to the present invention, or a kit according to the present invention, for use in medicine.

In one aspect, the present invention relates to a compound according to the present invention, or a kit according to the present invention for use in a method of diagnosis, prevention and/or treatment of a disease, such as a proliferative disease, a neurodegenerative disease, a viral infection, and/or a non-viral infection in a subject in need thereof.

In one preferred embodiment, the disease to be diagnosed, prevented and/or treated is associated with increased ROS levels in a diseased tissue compared to healthy tissue.

In one embodiment, the proliferative disease is cancer, preferably a cancer with solid tumor(s).

In one embodiment, the viral infection is an infection with human immunodeficiency virus (HIV), hepatitis B virus (HBV) and/or hepatitis C virus (HCV).

In one embodiment, the non-viral infection is a microbial infection.

In one embodiment, the subject is a mammal, such as an animal or a human, preferably a human.

In one aspect, the present invention relates to a method for the diagnosis, prevention and/or treatment of a disease, such as a proliferative disease, a neurodegenerative disease, a viral infection, and/or a non-viral infection, comprising the administration of a therapeutically effective amount of the compound according to the present invention, i.e. according to any one of the foregoing aspects and embodiments, or the kit according to the present invention, i.e. according to any one of the foregoing aspects and embodiments, to a subject in need thereof.

In one embodiment, the proliferative disease is cancer, preferably a cancer with solid tumor(s).

In one embodiment, the subject is a mammal, such as an animal or a human, preferably a human.

In one embodiment, the viral infection is an infection with human immunodeficiency virus (HIV), hepatitis B virus (HBV) and/or hepatitis C virus (HCV).

In one embodiment, the non-viral infection is a microbial infection.

In one embodiment, the compound or the kit is administered via oral, intravenous and/or intratumoral administration.

In one aspect, the present invention relates to the use of the compound according to the present invention, or the kit according to the present invention for the manufacture of a medicament for the diagnosis, prevention and/or treatment of a disease, such as a proliferative disease, a neurodegenerative disease, a viral infection, and/or a non-viral infection.

According to this aspect, the compound, the kit, the subject, the proliferative disease, the neurodegenerative disease, the viral infection and the non-viral infection are as defined herein.

### DETAILED DESCRIPTION

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

In one embodiment, the terms "subject" or "patient", as used herein, relates to a mammal, such as an animal or a human, preferably a human.

The term "drug", as used herein, refers to any chemical substance that is used to diagnose, treat, cure, or prevent a disease or condition in a subject. In the context of this invention, the drug is preferably selected from a group comprising
anti-cancer drugs, such as antimetabolites (e.g., methotrexate), alkylating drugs, such as cylcophosphamid, topoisomerase blockers, such as etoposide, enzymes, such as asparaginase, hormonal antagonists, such as tamoxifen, mitose blockers, such as vinca alkaloids, camptothecin, anthracyclines, such as doxorubicin, kinase inhibitors, such as erlotinib, gefitinib, imatinib, or lapatinib, hormones, such as estrogens or antiestrogens (e.g., estramustin), fulvestrant, gemcitabine, cytarabine, or proteasome inhibitors, such as bortezomib,
virustatics, such as nucleosides (e.g., emitricitabin), nucleotides, such as tenofovir, or non-nucleosides, such as nevirapine, ribavirin, ganciclovir, vidarabine, acyclovir, penciclovir, zalcitabine, protease inhibitors, such as lopinavir, blockers of the integrase, such as raltegravid, entry inhibitors, such as enfuvirtide, or amantadine, and
   antibiotics, such as betalactam antibiotics, penicillins, aminopenicillins, acylaminopenicillins, cephalosporins, cabapenems, monobactams, aminoglycosides, streptomycin, kanamycins, tetracyclines, macrolides and their analoga, streptogramines, oxazolidones, such as linezolid, benzochinon ansamycins, such as geldanamycin, chloramphenicol, fusidic acid, mupirocin or retapamulin, gyrase inhibitors, such as fluorochinolones, folic acid antagonists, such as sulfonamides, fidaxomicin, cyclic lipopeptids, such as daptomycin, polypeptides, such as polymyxin or colistin, and antitubercular medications, such as isoniazid, pyrazinamide, or ethambutol, RNA polymerase inhibitors, such as rifampicin, or clofazimin drugs, and
antimycotic drugs, such as azol-antimycotics (e.g. voriconazole or posaconazole), polyen-antimycotics, such as amphotericin B, nystatin, or natamycin, echinocandines, such as caspofungin, anidulafungin, or micafungin.

The term "functionalized compound", as used herein, refers to a compound having a general formula of any one of formulas (I) to (VII) wherein the choice of any of **R¹** to **R¹⁹** enables and/or influences the targeted control of ROS-sensitivity and/the finetuning thereof; and/or enables coupling of the boron-nitrogen heterocycles to various biological macromolecules, such as polyethylene glycol (PEG), peptides, proteins, such as antibodies, or enzymes, such as *Aplysia punctata* ink toxin (APIT).
For example, functionalization may be performed via coupling and/or attaching a drug or a reporter to at least one boron atom at position **R¹** of a compound having a general formula of any one of formulas (I) to (VII), wherein **R¹** either is or comprises said drug or said reporter, optionally wherein the drug or the reporter is coupled and/or is attached to at least one boron atom of the compound via a linker, wherein the linker is preferably a self-immolative linker. Additionally or alternatively, functionalization may also be performed by exchanging **R¹** or choosing a different **R¹** during synthesis of the compound. Functionalization at **R¹** also comprises the (further) functionalization of the linker, preferably the self-immolative linker, wherein the sensitivity of the compound to ROS, such as to H₂O₂, is dependent on steric and/or electronic characteristics of the linker, wherein the linker is preferably a self-immolative linker. Functionalization of the compound may also be performed by choice of **R²**, wherein the sensitivity of the compound to ROS, such as to H₂O₂, is dependent on **R²**, such as is dependent on steric and/or electronic characteristics of **R²**. Functionalization of the compound may also be performed by choice of any or R² to R⁹, and/or their subsequent modifications, which enables, for example, the coupling of the boron-nitrogen heterocycles to various biological macromolecules, such as polyethylene glycol (PEG), peptides, proteins, such as antibodies, or enzymes, such as *Aplysia punctata* ink toxin (APIT). In preferred embodiments of the present invention, functionalization of the compound at any of positions **R¹** to **R¹⁹** enables the targeted control of ROS-sensitivity, even more preferably, enables the finetuning of targeted control of ROS-sensitivity and also the degree of deactivation of the drug or the reporter.

In one embodiment, if the compound of the present invention is having a general formula of any one of formulas (II), (III), (V), the two instances of **R¹** within said compound are either be identical or the two instances of **R¹** are different from each other.

In one embodiment, both instances of **R¹** are or comprise the same drug or reporter.

In one embodiment, one **R¹** is or comprises a drug, wherein the other **R¹** is or comprises a reporter.

In one embodiment, one **R¹** is or comprises a first drug, wherein the other **R¹** is or comprises a second drug.

In one embodiment, one **R¹** is or comprises a first reporter, wherein the other **R¹** is or comprises a second reporter.

In one embodiment, when the drug or the reporter is coupled and/or is attached to at least one boron atom of the compound via a linker, the linker may be identical for both **R¹** or the linker is different for both instances of**R¹**.

In one embodiment, one **R¹** is a drug or reporter, wherein the other **R¹** comprises a drug or reporter.

In one embodiment, one **R¹** is or comprises a drug or reporter, wherein the other **R¹** is selected from a group comprising N(**R¹⁵**)₂, C₁-C₅ alkyl, C₁-C₂₀ alkoxycarbonyl, alkyne, aryl, alkene, and heteroaryl.

In one embodiment one **R¹** is or comprises a drug, wherein the other **R¹** is selected from a group comprising N(**R¹⁵**)₂, C₁-C₅ alkyl, C₁-C₂₀ alkoxycarbonyl, alkyne, aryl, alkene, and heteroaryl.

In one embodiment one R¹ is or comprises a reporter, wherein the other **R¹** is selected from a group comprising N(**R¹⁵**)₂, C₁-C₅ alkyl, C₁-C₂₀ alkoxycarbonyl, alkyne, aryl, alkene, and heteroaryl.

In one embodiment, the efficacy and/or tolerability of the compound according to the present invention has been confirmed by studies, such as clinical studies, e.g. animal studies.

The term "anti-cancer drugs", as used herein, refers to chemical substances or biological agents that are used to prevent, inhibit, or treat cancer. These drugs work through various mechanisms, such as killing cancer cells, inhibiting their growth and proliferation, or preventing metastasis.

The terms "cancer" and "cancer cells", as used herein, refer to any cells that exhibit uncontrolled growth in a tissue or organ of a multicellular organism. Particularly preferred cancers in context of the present invention are cancer with solid tumor(s), such as, however not limited to, breast cancer, lung cancer, prostate cancer, colorectal cancer, pancreatic cancer, liver cancer, kidney cancer, bladder cancer, ovarian cancer, melanoma, or sarcomas.

The term "antibiotics", as used herein, refers to chemical substances that are used to kill or inhibit the growth of bacteria. Antibiotics are used to treat bacterial infections and can be classified based on their mechanism of action, such as cell wall synthesis inhibitors, protein synthesis inhibitors, and nucleic acid synthesis inhibitors.

The term "virustatics", as used herein, refers to agents that inhibit the replication and spread of viruses within a host organism. These compounds do not necessarily kill the virus but rather prevent its proliferation, thereby helping to control viral infections and reduce disease severity.

The term "active drug", as used herein, refers to a pharmaceutical compound that is in a form capable of exerting its intended therapeutic effect. Active drugs interact with biological targets to produce a desired pharmacological response, such as alleviating symptoms, curing disease, or preventing illness. The term "inactive drug", as used herein, refers to a pharmaceutical compound that is in a form (partially) incapable of exerting its intended therapeutic effect. Inactive drugs may require activation through metabolic processes or may be rendered inactive due to chemical or physical modifications.

The term "aryl", as used herein, refers to an aromatic hydrocarbon group derived from an aromatic ring, such as phenyl or naphthyl. In the context of the present disclosure, the term "aryl" is to be understood that the hydrocarbon group may either be unsubstituted or substituted at one or more positions. Possible substitutions include, but are not limited to, halogen, C1-C5 alkyl, C1-C20 alkoxycarbonyl, alkyne, aryl, alkene, and heteroaryl.

The term "heteroaryl", as used herein, refers to an aromatic ring system in which one or more of the ring atoms is a heteroatom (such as nitrogen, oxygen, or sulfur) in place of a carbon atom. In the context of the present disclosure, the term "heteroaryl" is to be understood that the hydrocarbon group may either be unsubstituted or substituted at one or more positions. Possible substitutions include, but are not limited to, halogen, C1-C5 alkyl, C1-C20 alkoxycarbonyl, alkyne, aryl, alkene, and heteroaryl.

The term "alkene", as used herein, refers to a hydrocarbon containing at least one carbon-carbon double bond. In the context of the present disclosure, the term "alkene" is to be understood that the hydrocarbon group may either be unsubstituted or substituted at one or more positions. Possible substitutions include, but are not limited to, halogen, C1-C5 alkyl, C1-C20 alkoxycarbonyl, alkyne, aryl, alkene, and heteroaryl.

The term "alkyne", as used herein, refers to a hydrocarbon containing at least one carbon-carbon triple bond. In the context of the present disclosure, the term "alkyne" is to be understood that the hydrocarbon group may either be unsubstituted or substituted at one or more positions. Possible substitutions include, but are not limited to, halogen, C1-C5 alkyl, C1-C20 alkoxycarbonyl, alkyne, aryl, alkene, and heteroaryl.

The term "alkoxycarbonyl", as used herein, refers to a functional group with the structure - COOR, where R is an alkyl group. In the context of the present disclosure, the term "alkoxycarbonyl" is to be understood that the group may either be unsubstituted or substituted at one or more positions. Possible substitutions include, but are not limited to, halogen, C1-C5 alkyl, C1-C20 alkoxycarbonyl, alkyne, aryl, alkene, and heteroaryl.

The term "reporter", as used herein, refers to a molecule or substance used to indicate the presence, quantity, or activity of another substance or biological process. In the context of this invention, the reporter is preferably selected from a group comprising
fluorescence dyes, such as near infrared (NIR) dyes, e.g. resorufin or 2-[2-(2,3-Dihydro-6-hydroxy-1*H*-xanthen-3-yl)ethenyl]-1-ethyl-3,3-dimethyl-3*H*-indolium,
diagnostic macromolecules,
mass reporters, and
chemoluminescence dyes.

The term "fluorescence dye(s)", as used herein, refers to chemical compounds that can absorb light at a specific wavelength and subsequently emit light at a longer wavelength. These dyes are used extensively in biological and chemical assays to label and visualize molecules, cells, or tissues, and are valuable tools in techniques such as fluorescence microscopy, flow cytometry, and fluorescence in situ hybridization (FISH). Examples for suitable fluorescence dyes are near infrared (NIR) dyes.

The term "near infrared (NIR) dyes", as used herein, refers to a specific category of fluorescence dyes that emit light in the near-infrared region of the electromagnetic spectrum, typically between 700 nm and 900 nm. These dyes are particularly useful in biological imaging and diagnostic applications because NIR light penetrates tissues more deeply and with less scattering than visible light, resulting in clearer images. Examples for NIR dyes are resorufin or 2-[2-(2,3-Dihydro-6-hydroxy-1*H*-xanthen-3-yl)ethenyl]-1-ethyl-3,3-dimethyl-3*H-*indolium.

The term "diagnostic macromolecules", as used herein, refers to large molecules, such as proteins, nucleic acids, or polysaccharides, that are used in diagnostic applications to detect, quantify, or monitor specific biological markers or disease states. These macromolecules can include antibodies, aptamers, enzymes, and other biologically active molecules that interact with target analytes to provide diagnostic information.

The term "mass reporters", as used herein, refers to molecules or entities that are used to provide a detectable mass signal in mass spectrometry-based assays. Mass reporters are designed to produce a unique and identifiable mass signature that can be easily distinguished from other components in the sample, thereby enabling precise and accurate measurement of the target compound. They are particularly useful in applications such as quantitative proteomics, drug metabolism studies, and biomarker discovery, where high sensitivity and specificity are essential for detecting and analyzing specific molecules within a complex mixture.

The term "chemoluminescence dyes", as used herein, refers to chemical compounds that emit light as a result of a chemical reaction. These dyes are used in various analytical and diagnostic applications, including immunoassays, nucleic acid detection, and reporter gene assays, where the emitted light serves as a measurable signal to indicate the presence or activity of a target analyte or biological process.

The term "active reporter", as used herein, refers to a reporter molecule that is in a form capable of producing a detectable signal or response. Active reporters are used in various assays and imaging techniques to monitor biological processes, track molecules, or quantify analytes. The term "inactive reporter", as used herein, refers to a reporter molecule that is in a form (partially) incapable of producing a detectable signal or response. Inactive reporters may require activation through specific triggers or may be rendered inactive due to chemical or physical modifications.

The term "steric characteristics", as used herein, refers to the spatial properties and physical dimensions of a molecule that influence its ability to interact with other molecules. These characteristics include the size, shape, and bulkiness of the molecule. In the case of "steric characteristics of a linker" these properties can affect the accessibility and binding of the linked molecules to their respective targets. Steric characteristics play a crucial role in determining the overall conformation and flexibility of the linker, as well as its potential to cause steric hindrance or facilitate favorable molecular interactions.

The term "electronic characteristics", as used herein, refers to the distribution of electrons within a molecule and the resulting chemical reactivity and interaction potential of the molecule. These characteristics include the presence of electron-donating or electron-withdrawing groups, the overall polarity of the molecule, and its ability to participate in electronic interactions such as hydrogen bonding, dipole-dipole interactions, and π-π stacking. Electronic characteristics influence the stability, reactivity, and binding affinity of the molecule. In the case of "electronic characteristics of a linker" these characteristics may also influence its ability to modulate the electronic environment of the linked molecules.

The term "sterically demanding", as used herein, refers to a chemical entity or moiety that occupies significant spatial volume and exerts considerable steric hindrance on its surroundings. Such entities can interfere with the binding or interaction of other molecules due to their larger size and increased bulk.

The term "sterically less demanding", as used herein, refers to a chemical entity or moiety that occupies less spatial volume and exerts minimal steric hindrance on its surroundings. Such entities are less likely to interfere with the binding or interaction of other molecules due to their smaller size and reduced bulk.

The term "reactive oxygen species (ROS)", as used herein, refers to highly reactive chemicals formed from oxygen. These species include, but are not limited to, peroxides, superoxide, hydroxyl radical, and singlet oxygen. ROS are known to play a dual role as both toxic byproducts of cellular metabolism and as signaling molecules that modulate various physiological processes. ROS in the context of the present invention preferably refers to H₂O₂.

The term "increases the level of reactive oxygen species (ROS)", as used herein, such as in "a peptide/ a protein/ an enzyme which increases the level of reactive oxygen species (ROS)" refers to any peptide, protein, or enzyme that facilitates the production or accumulation of reactive oxygen species within a biological system. This may occur through various mechanisms, such as the activation of specific metabolic pathways, the catalysis of redox reactions, or the disruption of cellular antioxidant defenses.

The terms "release of an (active) drug", "release of an (active) reporter" and the like, as used herein, refers to the process by which a pharmaceutical ingredient or a reporter molecule is liberated from a formulation or a conjugate, preferably in a biologically active form.

The term "is coupled to", as used herein, refers to a chemical or physical linkage between two entities, which may involve covalent bonds, ionic bonds, hydrogen bonds, or other forms of association or which may involve the connection of two entities via a linker, preferably a self-immolating linker. This coupling can be designed to be stable or cleavable or self-immolating under specific conditions, depending on the intended application.

The term "is attached to", as used herein, refers to the state in which one entity is connected to another, either through direct bonding or via a linker, preferably a self-immolating linker. This attachment can influence the properties and functions of the entities involved, and can be reversible or irreversible based on the nature of the bond or linkage.

The term "self-immolative linker", as used herein, refers to a type of chemical linker that undergoes a spontaneous cleavage upon triggering, leading to the release of the attached molecule. This triggering can be initiated by various stimuli, such as the presence of ROS, enzymatic action, light, or changes in pH, preferably the presence of ROS, and the self-immolative nature ensures that the linker disassembles to a large extent, preferable the linker disassembles completely, releasing the active agent in its original form.

The term "alkyl-based linker", as used herein, refers to a linker composed predominantly of alkyl groups. These linkers are characterized by their flexibility and hydrophobic nature, and are often used to connect molecules in a way that allows for rotational freedom and minimal steric hindrance.

The term "aryl-based linker", as used herein, refers to a linker composed predominantly of aryl groups, which are aromatic hydrocarbons. These linkers are characterized by their rigidity and planarity, and can impart specific electronic and steric properties to the conjugated molecules, influencing their behavior and interactions.

The term "pharmaceutically acceptable salt thereof", as used herein, refers to a salt form of a compound that is suitable for use in pharmaceutical applications, ensuring safety and efficacy. These salts may be formed by the reaction of the compound with pharmaceutically acceptable acids or bases, and are used to enhance the solubility, stability, and bioavailability of the compound.

The term "chemically modified" such as in "the compound is chemically modified", as used herein, refers to any alteration made to the chemical structure of a compound, which may involve the addition, deletion, or substitution of atoms or functional groups. Such modifications can be made to improve the compound's pharmacokinetic properties, reduce toxicity, enhance efficacy, or introduce new functionalities.

The term "Aplysia punctata ink toxin (APIT)", as used herein, refers to a specific toxin derived from the ink of the sea hare *Aplysia punctata,* known for its biological activity. This toxin has been studied for its potential therapeutic applications, including its ability to modulate immune responses, inhibit microbial growth, or induce cell death in certain cancer cells. Preferably, said APIT comprises or consists of an amino acid sequence which is at least 90% identical, preferably at least 95% identical, more preferably at least 98% identical, even more preferably at least 99% identical, optionally identical, to the amino acid sequence of SEQ ID NO. 1.

The term "drug delivery system (DDS)", as used herein, refers to a formulation designed to transport a pharmaceutical compound to its target site within the body in a controlled manner. Drug delivery systems aim to improve the efficacy, safety, and bioavailability of drugs by optimizing their release profile, targeting specific tissues or cells, and minimizing side effects.

The term "antibody", as used herein, is used interchangeably with the term "immunoglobulin", and refers to a glycoprotein comprising at least two heavy (H) chains and two light (L) chains inter-connected by disulfide bonds. The term also includes all recombinant forms of antibodies, e.g. antibodies expressed in prokaryotes, unglycosylated antibodies and derivatives as described below. There are five different types of heavy chains, which define antibody isotypes of different functional activity: IgM, IgD, IgG, IgA and IgE. Each heavy chain comprises a heavy chain variable region (abbreviated herein as V_{H} region) and a heavy chain constant region. Each light chain comprises a light chain variable region (abbreviated herein as V_{L} region) and a light chain constant region. The V_{H} and V_{L} regions can be further subdivided into regions of hypervariability, termed complementarity determining regions (CDR), interspersed with regions that are more conserved, termed framework regions (FR). The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The terms "diagnosis" or "diagnostic", or similar expressions, as used herein, refer to identifying the presence or absence, or identifying the nature of a condition, preferably a pathologic condition, in a subject. Diagnostic methods differ in their sensitivity and specificity. While a particular diagnostic method may not provide a definitive diagnosis of a condition, it suffices if the method provides a positive indication that aids in diagnosis.

The term "prevention of a disease", as used herein, refers to measures or interventions such as a process of providing a subject with a pharmaceutical treatment, e.g., the administration of a drug, such that at least one symptom of the disease prevented from occurring.

The term "treatment of a disease", as used herein, refers to therapeutic interventions such as the process of providing a subject with a pharmaceutical treatment, e.g., the administration of a drug, such that at least one symptom of the disease is decreased. Treatment of a disease is aimed at improving, curing, mitigating, or managing the symptoms or progression of a disease in a subject.

### BRIEF DESCRIPTION OF THE FIGURES

The present invention is now further described by reference to the following figures.

All methods mentioned in the figure descriptions below were carried out as described in detail in the examples.
**Figure 1****.** Reaction of the BNN heterocycles to phenols (R = aromatic).
**Figure 2****.** Release of the drug (represented by grey circles) in the presence of H₂O₂ illustrated using a example structure.
**Figure 3****.** HPLC stability measurements of selected BNN heterocycles upon reaction with 1000 equivalents of ROS.
**Figure 4****.** HPLC stability measurement of selected BNN heterocycles when reacted by APIT and lysine.
**Figure 5****.** HPLC stability measurements of selected BNN heterocycles upon reaction with 100 equivalents of ROS.
**Figure 6****.** HPLC stability measurements of a BNN-diazaborole and a NBN-diazaborole upon reaction with 1000 equivalents of ROS.
**Figure 7****.** HPLC stability measurements of BNN heterocycles demonstrate the influence of the ring size of the heterocycles on the ROS-sensitivity.
**Figure 8****.** Example molecule for ROS-sensitive drug release systems with Camptothecin.
**Figure 9****.** Example molecule for ROS-sensitive drug release systems with maleimidohexanoic acid.
**Figure 10****.** HPLC stability measurement of a chlorambucil-modified BNN, when reacted with 1000 equivalents of ROS.
   **A)** Exemplified reaction mechanism. **B)** The chlorambucil-modified BNN (compound A) and chlorambucil (compound B) exhibit distinct retention times. **C)** Stability of compound A and compound B over time.
**Figure 11****.** Synthesis of compounds of type **F1** or **G1** without further functionalization at the N atom (PG = protecting group, LG = leaving group, Hal = halogen, Tf = triflate, Ts = tosylate) illustrated using an example structure.
**Figure 12****.** Synthesis of compounds of type **SS** post-synthetic functionalization at the N atom (PG = protecting group, LG = leaving group, Hal = halogen, Tf = triflate, Ts = tosylate, M = metal) illustrated using example structures.
In the following, reference is made to the examples, which are given to illustrate, not to limit the present invention.

### EXAMPLES

ROS, such as H₂O₂, are physiological transmitters, wherein for the levels of ROS pathophysiological differences exist between healthy and diseased tissues. Therefore, the present application aims on exploiting their potential for targeted and controllable drug or reporter release. To this end, the present invention provides compounds comprising boron-nitrogen heterocycles which may be differently functionalized and which can be used both therapeutically and diagnostically.

### Example 1

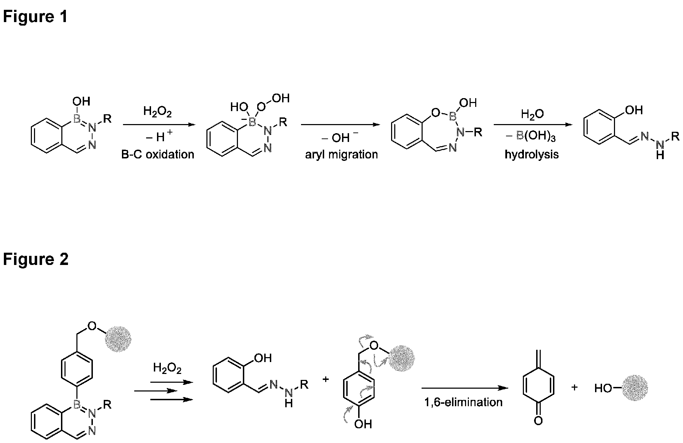

In the compounds for drug delivery systems according to the invention, increased concentrations of ROS, in particular H₂O₂, are utilized in diseased tissue. In order to utilize the increased H₂O₂ concentrations, various BNN heterocycles were prepared. The BNN heterocycles according to the invention react in the presence of H₂O₂ to form phenols (**Figure 1**). This reactivity can be used, for example, to determine the hydrogen peroxide level of mitochondria in living cells or oxidative stress *in vivo* using fluorescence.

According to the present invention, the phenolic and boronic acid derivatives formed by the H₂O₂-induced reaction in the compounds shown subsequently react further and thus release the desired active ingredient in a cascade reaction or already during the decomposition reaction (**Figure 2**).

For the compounds described herein, ROS, such as H₂O₂, are used as a trigger to release the active drug or the active reporter. An increased ROS concentration, such as an increased H₂O₂ concentration compared to healthy tissue is therefore necessary for effective drug release in diseased tissue. The compounds according to the present invention are therefore particularly suitable for use in diseases in which an increased ROS level can be observed, such as proliferative diseases, a neurodegenerative diseases, viral infections, or non-viral infections.

### Example 2

Molecules were produced which can (partially) inactivate the active substance by coupling (self-immolative prodrug), as well as molecules which can additionally be linked to macromolecules by modifying the naphthalenoid benzene ring. Such a coupling to macromolecules can, among other things, enable a targeted distribution of the construct in the body (self-immolative linker).

One unique feature of this invention is at least the targeted control of ROS-sensitivity and thus also the degree of deactivation through the functionalization of the boron-nitrogen heterocycle. For example, this may involve the targeted control of ROS-sensitivity and thus also the degree of deactivation through the functionalization of the self-immolative linker on the boron or the adjacent *α*-nitrogen atom. In a series of experiments, the structure-activity principles (SAR) between electronics and sterics of the boron and nitrogen substituents were determined. Without wishing to be bound by any theory, two parameters are proposed for fine tuning, depending on the magnitude of the ROS concentrations present:
(1) for increased ROS gradients: manipulating the steric demand on the boron atom, e.g. via ortho-substitution with a suitable residue (alkyl group, aryl group, halogen) / by anellation of the aryl substituent in the form of a naphthalinoid or anthranoid system or
(2) for lower ROS gradients: modifying the steric demand on the neighboring α-nitrogen atom via analogous manipulations.

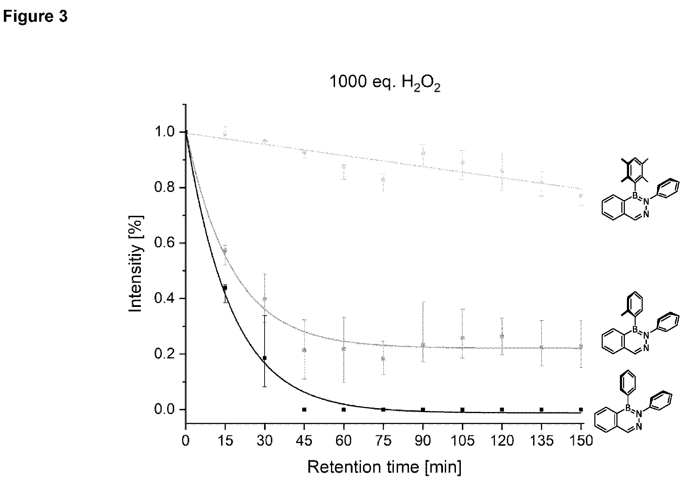

**Figure 3** shows an example of the stability of three selected BNN heterocycles with variable steric demand at the boron atom towards 1000 equivalents of ROS. A stabilization of the heterocycles is achieved by successively increasing the steric demand by introducing *ortho-*methyl groups.

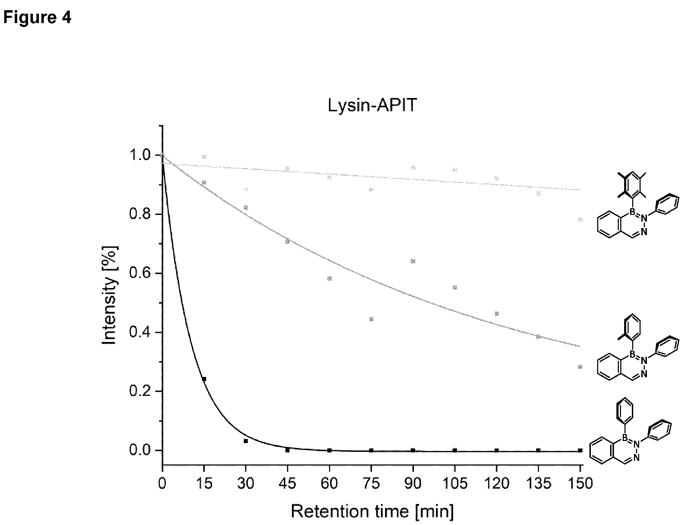

The same effect can be observed when the heterocycles are incubated with the protein APIT and the amino acid lysine (see **Figure 4**). The enzyme APIT is able to deaminate lysine and generate ROS in the process. These ultimately attack the heterocycles as described above.

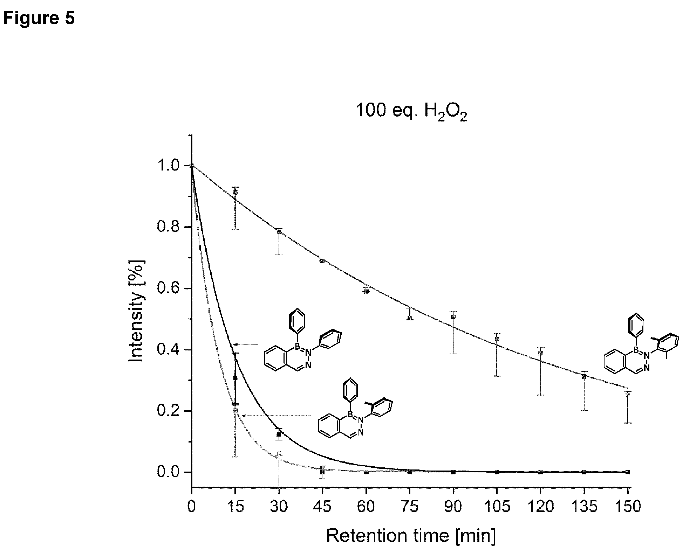

Similarly, **Figure 5** shows the stability with variable steric demand on the *α*-nitrogen atom compared to 100 equivalents of ROS. Increasing the steric demand by introducing two *ortho-*methyl groups also stabilizes the heterocycles.

Summary of the underlying experimental procedure:
For the reaction of the compounds with hydrogen peroxide in **Figure 3** a solution (1000 equivalents) was prepared with 100 µL 3% hydrogen peroxide and 780 µL millipore water. For **Figure 5** a 100 equivalents hydrogen peroxide solution was prepared from 10 µL 3% hydrogen peroxide and 870 µL millipore water.

A 10 mM stock solution was prepared in acetonitrile. 10 µL of the stock solution was diluted with 990 µL 1× phosphate buffered saline to get a concentration of 100 µM. For the reaction with H₂O₂, 250 µL of the 100 µM solution was mixed with 250 µL of the respective hydrogen peroxide solution so that the final sample concentration was 50 µM.

For the reaction of the compounds with lysine and Aplysia punctata ink toxin (APIT) (**Figure 4**), a 100 mM lysine solution was prepared by mixing 0.365 g lysine hydrochloride with 20 mL 1× phosphate buffered saline. Then a 10 mM stock solution was prepared in acetonitrile. 10 µL of the stock solution was diluted with 990 µL 1× phosphate buffered saline to get a concentration of 100 µM. For the reaction, 250 µL of the 100 µM solution was mixed with 250 µL lysine solution and 1.7 µL APIT, so that the final sample concentration was 50 µM.

The analytical HPLC study was performed with an Agilent 1260 infinity II HPLC (Agilent Technologies Inc., Waldbronn, Germany), using a Zorbax XDC-18 analytical 4.6 × 150 mm LC column (Agilent Technologies Inc., Waldbronn, Germany). The instrument was equipped with a variable wavelength detector (G7114A, Agilent 1260 DAD WR), an automatic vial sampler (G7129C, Agilent), a flexible pump (G7104C, Agilent pump (G7104C, Agilent) and 3 multi-column ovens (G7116A, Agilent). For the high-performance liquid chromatography procedure, the mobile phase C was 0.1% formic acid in millipore water. Mobile phase D was acetonitrile with 0.1% formic acid, the flow rate was set to 1 mL/min, the injection volume was 20 µL, and the wavelength of the detector was set to λ [nm] = 254, 278, 300, 310 and 350. The gradient was held at 5% D and 95% C for 2 minutes, increased to 100% D and 0% C within 7 minutes, held for 2 minutes, then reduced back to 5% D and 95% C within 2 minutes and held for 2 minutes.

### Example 3

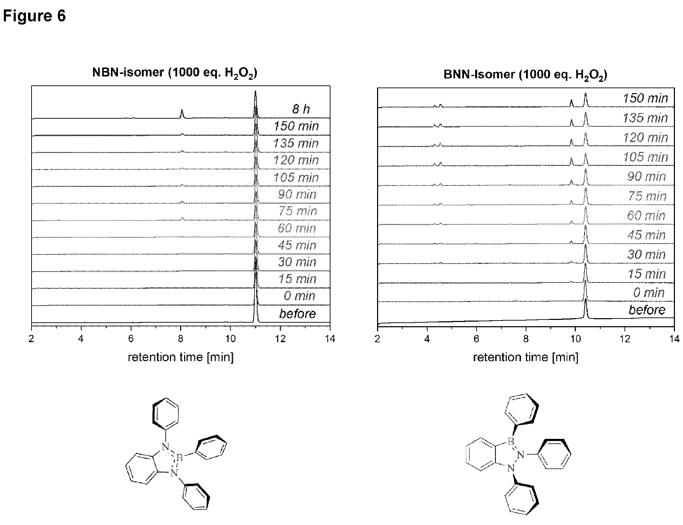

Besides the substituents, the ROS-sensitivity can also be further adjusted by the ring size and/or the placement of the heteroatoms in the BNN heterocycle. **Figure 6** shows the stability of two diazaborole isomers, whereby the NBN isomer is clearly stabilized in comparison to the BNN isomer.

Moreover, the compounds of the present invention can be used together with various macromolecules that produce ROS, e.g. H₂O₂, such as Aplysia punctata ink toxin (APIT). Various strategies are encompassed by the present invention. On the one hand, the compounds can be administered in combination with APIT, on the other hand, it is possible to directly or indirectly attach APIT to a compound having a general formula of any of the formulas (I) to (VII) at any of R² to R⁹, preferably at any of R³ to R⁹.

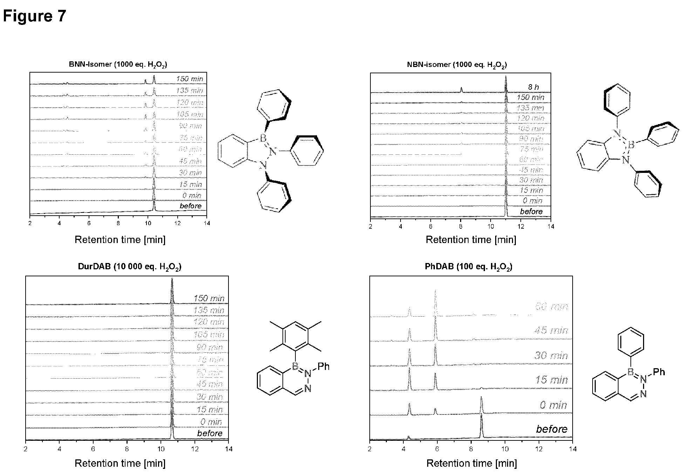

The influence of the ring size of the BNN heterocycle has also been tested. **Figure 7** demonstrates that compounds comprising a 5-membered BNN heterocycle exhibit a higher stability compared to compounds comprising a 6-membered BNN heterocycle. These data demonstrate that ROS-sensitivity can be adjusted by the choice of the ring size of the BNN heterocycles or the positioning of the heteroatoms within the heterocycle.

### Example 4

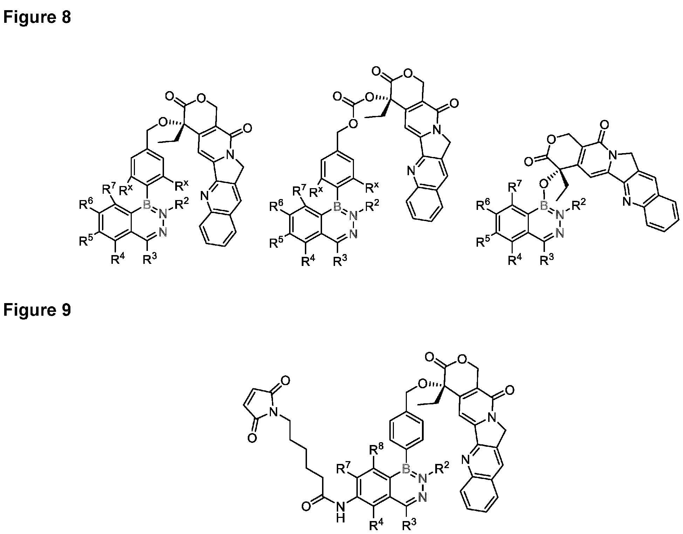

According to the present invention, it is further possible to couple and/or attach an active drug or reporter to a compound having a general formula of any of the formulas (I) to (VII). As exemplified in **Figure 8****,** a drug, such as the commercially available chemotherapeutic agent camptothecin may be coupled to the compound via an ether bond (**Figure 8****,** left and middle) or via an ester bond (**Figure 8****,** right). In the presence of H₂O₂ the active drug is released from the compound.

Moreover, the compounds of the present invention having a general formula of any of the formulas (I) to (VII) may further be modified at one or more of position of R²⁻⁹, wherein said modification(s) enable the attachment of at least one further moiety. **Figure 9** illustrates the coupling of maleimidohexanoic acid to a compound via a NH₂ group. This functionalization makes it possible to connect the molecule with macromolecules via free thiols.

### Example 5

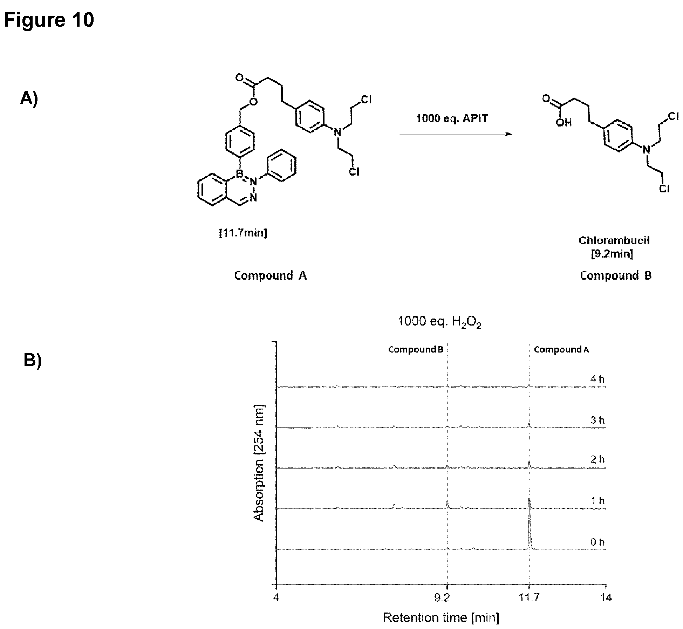

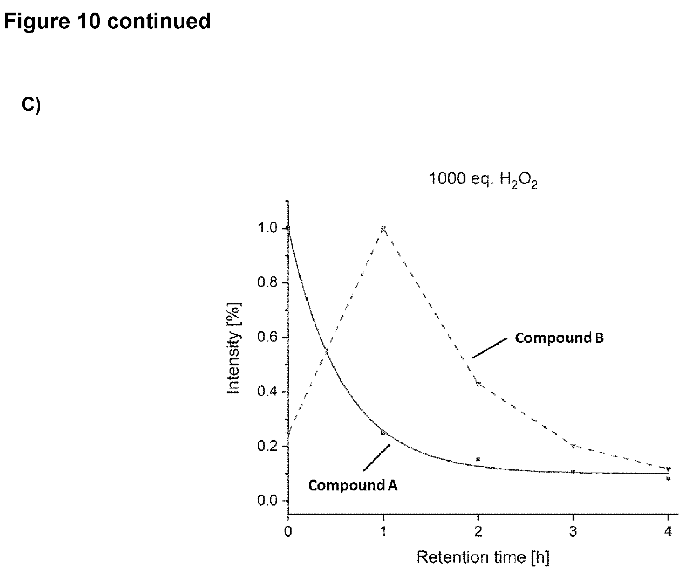

The release of an active agent from a heterocycle is shown in **Figure 10****.** First, a heterocycle is modified by esterification with the chemotherapeutic agent chlorambucil. This molecule is then reacted with 1000 eq. H₂O₂ and its absorption measured over 4 h on the HPLC. The modified heterocycle elutes after a retention time of 11.7 min, while chlorambucil elutes after 9.2 min (**Figure 10B**). Initially, only the modified heterocycle is detected in the chromatogram. However, after an incubation time of one hour, a clear chlorambucil signal can be seen, while the signal of the modified heterocycle is reduced (**Figure 10C**).

### Example 6

### Synthesis of compounds according to the present invention

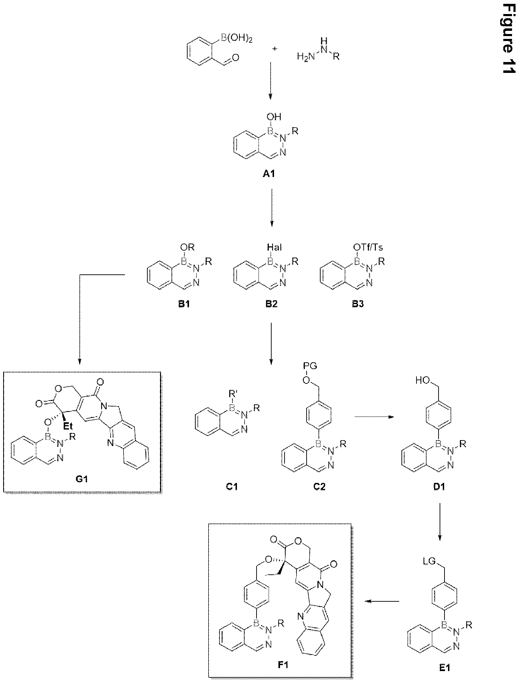

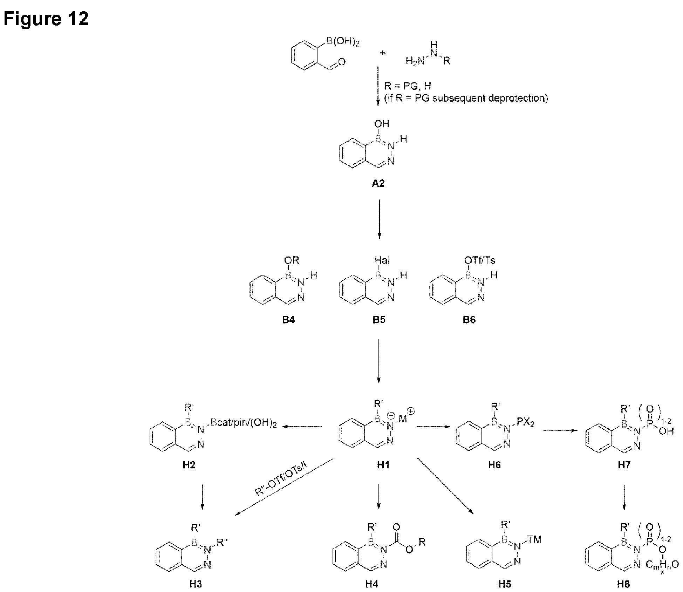

The preparation of the compounds described herein is possible via several synthesis routes. Due to the good yield and simple isolation, the compounds described in the present invention are ring-closed to the BNN heterocycle **A** in the first step starting from a 2-formylphenyl boronic acid derivative by reaction with a hydrazine derivative. The boronic acid derivative used can also be a thiophene, pyrrole or furan derivative. The hydrazine used can have various substituents on the *β*-nitrogen atom (e.g. H, alkyl, aryl, hydroxy, thiol, COOR, amine protecting group; **Figures 11** and **12**) and optionally be present as a hydrochloride salt.

In the next step, if no further nitrogen functionalization is required, the boric acid function is replaced by a better leaving group such as a boronic acid ester **B1** (R = silyl, alkyl, aryl), a halogen **B2** or a triflate/tosylate **B3.** Subsequently, the boron atom is functionalized by reaction with a trimethylsilyl compound in a TMSCl (trimethylsilyl chloride) elimination reaction or, alternatively, a suitable nucleophile to **C1** or **C2** in a metathesis reaction. Suitable carbon nucleophiles for this purpose include alkali metal organyls (R'-Li, R'-Na, R'-K), Grignard reagents (R'MgX), organocuprates or organocer compounds. In the case of a molecule such as **C1,** a deprotection (**D1**) and subsequent leaving group transformation (**E1**) is then carried out. Final reaction with deprotonated camptothecin can yield **F1**-type compounds (**Figure 11**).

Alternatively, a conjugate of the form **G1** can also be obtained by direct reaction of **B1, B2** or **B3** with deprotonated camptothecin in a metathesis reaction (**Figure 11**).

For an additional functionalization of the N1 atom, either the parent hydrazine N₂H₄ or an N-protected hydrazine with deprotection after ring closure reaction is used to obtain the parent compound **A2** (**Figure 12**). Here, the boric acid function is also replaced by a more suitable leaving group such as a boronic acid ester **B4** (R = silyl, alkyl, aryl), a halogen **B5** or a triflate/tosylate **B6.** Subsequently, the boron atom is functionalized by reaction with a suitable nucleophile in a metathesis reaction with simultaneous deprotonation to **H1.** Suitable carbon nucleophiles for this purpose include alkali metal organyls (R'-Li, R'-Na, R'-K), Grignard reagents (R'MgX), organocuprates or organocer compounds.

The metallated diazaborinines of type **H1** can be isolated and subsequently further functionalized at the N1 atom. By reaction with suitable organoboron compounds, compounds of type **H2** can be obtained which, for example, allow the introduction of a substituent R" (R" = aryl, OR, halogen, C₁-C₅ alkyl, C1-C4 alkoxy, C1-C20 alkoxycarbonyl) by transition metal-catalyzed cross-coupling reactions (**H3**). Similarly, an R"-substituted diazaborinine **H3** can be obtained by reaction of H1 with an organotriflate, -tosylate, or -iodide. By reaction with a carboxylic acid source such as carbon dioxide, carboxylic acid amides (**H4**) are obtained. Compounds of type **H5** can be obtained by reaction with suitable transition metal precursors. The reaction with phosphorus halides (**H6**) with subsequent hydrolysis yields phosphoric acid derivatives (**H7**) which are subsequently functionalized with sugar molecules (H**8**). Depending on the carbon nucleophile used, the N-functionalization can be followed by camptothecin loading analogous to **Figure 11****.**

### Example 7

### Conclusion

The above examples convincingly and surprisingly show that the boron-nitrogen heterocycles of the present invention are highly suitable for the targeted and controllable release of an active drug or an active reporter in diseased tissues with increased ROS concentrations. In particular, in a series of experiments, the structure-activity principles (SAR) between electronics and sterics of the boron and nitrogen substituents were determined. By functionalizing at the boron atom, using different ring sizes and BNN positions in the ring, as well as independent functionalization at the nitrogen atom, fine adjustment of the ROS-sensitivity of the compounds is made possible by steric and electronic factors. This fine adjustment enables a targeted and controllable release of the active substance/dye/fluorophore in tissues in which the ROS concentration is upregulated due to various diseases. First, a ROS-induced reaction enables the conversion of the BNN heterocycles to phenylboronic acid derivatives and phenol derivatives. By prior attachment of cascade linkers to the boron or nitrogen atom or direct attachment of the active substance/dye/fluorophore to the boron or nitrogen atom, the active substance is then released unchanged (self-immolative prodrug). Possible functionalization on the aromatic compound enables coupling of the boron-nitrogen heterocycles to various biological macromolecules, such as polyethylene glycol (PEG), peptides, proteins, such as antibodies, or enzymes, such as *Aplysia punctata* ink toxin (APIT). This enables, among other things, an extension of the half-life or a targeted distribution of the construct in the body. In this case, the boron-nitrogen heterocycles may act as linkers between the macromolecule and the active substance (self-immolative linkers). In particular, substances that are used for the treatment of diseases in which the concentration of ROS is upregulated are to be used as drugs or active substances.

## Claims

1. A compound having a general formula of any one of formulas (I) to (VII) 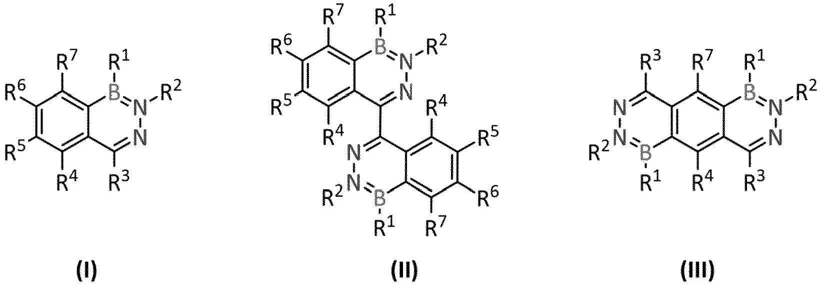 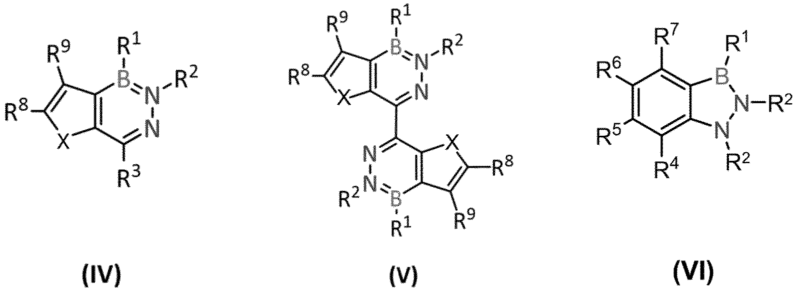 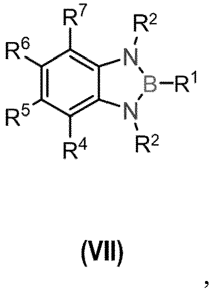 wherein
**R¹** is or comprises, at each occurrence, independently a drug or a reporter, or is, at each occurrence, independently selected from a group comprising N(**R¹⁵**)₂, C₁-C₅ alkyl, C₁-C₂₀ alkoxycarbonyl, alkyne, aryl, alkene, and heteroaryl, wherein at least one **R¹** is or comprises a drug or a reporter,
optionally wherein the drug or the reporter is coupled and/or is attached to at least one boron atom of the compound via a linker, wherein the linker is preferably a self-immolative linker, such as an alkyl-based linker or an aryl-based linker, and/or a linker comprising an ether and/or an ester group,
**R²** to **R⁹** are, at each occurrence, independently selected from a group comprising H, OH, O**R¹⁵**, N(**R¹⁵**)₂, NO₂, SO₂, P(**R¹⁶**)₃₋₅, halogen, C₁-C₅ alkyl, C₁-C₂₀ alkoxycarbonyl, alkyne, aryl, alkene, and heteroaryl, wherein said aryl or said heteroaryl is optionally substituted with one or more of **R¹⁸,** preferably one or two of **R¹⁸,**
**X** is O, N**R¹⁵**, S, SO, or SO₂,
**R¹⁵** is, at each occurrence, independently selected from a group comprising H, OH, halogen, C₁-C₁₀ alkyl, aryl, heteroaryl, and (C=O)**R¹⁹**,
**R¹⁶** is, at each occurrence, independently O or O**R¹⁷**,
**R¹⁷** is, at each occurrence, independently selected from a group comprising P, C₁-C₁₀ alkyl, C₁-C₁₀ aryl, C₁-C₁₀ heteroaryl, and ribose,
**R¹⁸** is, at each occurrence, independently H or C₁-C₅ alkyl, and
**R¹⁹** is, at each occurrence, independently C₃-C₁₀ alkyl, optionally substituted with a maleimide group,
or a pharmaceutically acceptable salt thereof.

2. The compound according to claim 1, wherein at least one boron atom or nitrogen atom of the compound is chemically modified.

3. The compound according to claim 1 or 2, wherein the linker, preferably the self-immolative linker, has a general formula of any one of formulas (A) to (F): 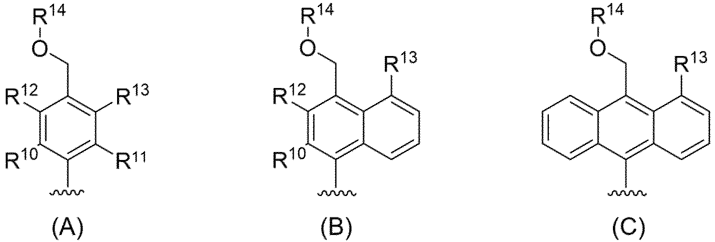 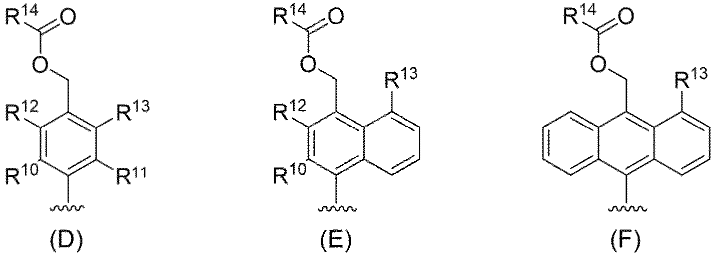 wherein **R¹⁰, R¹¹, R¹²,** and **R¹³** are each independently selected from a group comprising H, OH, O**R¹⁵**, halogen, C₁-C₁₀ alkyl, aryl, and heteroaryl, and wherein **R¹⁴** is or comprises a drug or a reporter.

4. The compound according to any one of claims 1 to 3, wherein the compound comprises one or more further moieties,
wherein the one or more further moiety is preferably a polymer, more preferably polyethylene glycol (PEG),
wherein said polymer is preferably attached to the compound having a general formula of any one of formulas (I) to (VII) at any of **R²** to **R⁹**, more preferably at any of **R³** to **R⁹,**
and/or
wherein the one or more further moiety is preferably a peptide or a protein, such as a peptide or a protein which increases the level of reactive oxygen species (ROS), for example an antibody or an enzyme which increases the level of ROS, preferably an L-amino acid oxidase, such as Aplysia punctata ink toxin (APIT), which comprises or consists of an amino acid sequence which is at least 90% identical, preferably at least 95% identical, more preferably at least 98% identical, even more preferably at least 99% identical, optionally identical, to the amino acid sequence of SEQ ID NO. 1,
wherein said peptide or protein is preferably covalently attached to the compound having a general formula of any one of formulas (I) to (VII) at any of **R²** to **R⁹,** more preferably at any of **R³** to **R⁹.**

5. The compound according to any one of claims 1 to 4, wherein **R¹** or **R¹⁴** is or comprises a drug which is selected from a group comprising
anti-cancer drugs, such as antimetabolites (e.g., methotrexate), alkylating drugs, such as cylcophosphamid, topoisomerase blockers, such as etoposide, enzymes, such as asparaginase, hormonal antagonists, such as tamoxifen, mitose blockers, such as vinca alkaloids, camptothecin, anthracyclines, such as doxorubicin, kinase inhibitors, such as erlotinib, gefitinib, imatinib, or lapatinib, hormones, such as estrogens or antiestrogens (e.g., estramustin), fulvestrant, gemcitabine, cytarabine, or proteasome inhibitors, such as bortezomib,
virustatics, such as nucleosides (e.g., emitricitabin), nucleotides, such as tenofovir, or non-nucleosides, such as nevirapine, ribavirin, ganciclovir, vidarabine, acyclovir, penciclovir, zalcitabine, protease inhibitors, such as lopinavir, blockers of the integrase, such as raltegravid, entry inhibitors, such as enfuvirtide, or amantadine, and
antibiotics, such as betalactam antibiotics, penicillins, aminopenicillins, acylaminopenicillins, cephalosporins, cabapenems, monobactams, aminoglycosides, streptomycin, kanamycins, tetracyclines, macrolides and their analoga, streptogramines, oxazolidones, such as linezolid, benzochinon ansamycins, such as geldanamycin, chloramphenicol, fusidic acid, mupirocin or retapamulin, gyrase inhibitors, such as fluorochinolones, folic acid antagonists, such as sulfonamides, fidaxomicin, cyclic lipopeptids, such as daptomycin, polypeptides, such as polymyxin or colistin, and antitubercular medications, such as isoniazid, pyrazinamide, or ethambutol, RNA polymerase inhibitors, such as rifampicin, or clofazimin drugs, and
antimycotic drugs, such as azol-antimycotics (e.g. voriconazole or posaconazole), polyen-antimycotics, such as amphotericin B, nystatin, or natamycin, echinocandines, such as caspofungin, anidulafungin, or micafungin.

6. The compound according to any one of claims 1 to 5, wherein **R¹** or **R¹⁴** is or comprises a reporter which is selected from a group comprising
fluorescence dyes, such as near infrared (NIR) dyes, e.g. resorufin or 2-[2-(2,3-Dihydro-6-hydroxy-1*H*-xanthen-3-yl)ethenyl]-1-ethyl-3,3-dimethyl-3*H*-indolium,
diagnostic macromolecules,
mass reporters, and
chemoluminescence dyes.

7. The compound according to any one of claims 1 to 6, wherein the coupling and/or attaching of the drug or the reporter to a boron-nitrogen heterocycle backbone of a compound having a general formula of any one of formulas (I) to (VII) inactivates the drug or the reporter, such as partially and/or temporarily inactivates the drug or the reporter, optionally wherein an active drug and/or an active reporter is released in presence of a reactive oxygen species (ROS), such as hydrogen peroxide (H₂O₂).

8. The compound according to any one of claims 1 to 7, wherein the sensitivity of the compound to ROS, such as to H₂O₂, is dependent on steric and/or electronic characteristics of the linker, wherein the linker is preferably a self-immolative linker, and wherein the release of an active drug and/or an active reporter in presence of ROS, such as H₂O₂, is faster when the linker is (a) small and/or sterically less demanding compared to the release of an active drug and/or an active reporter in presence of ROS, such as H₂O₂, when the linker is (b) large and/or sterically demanding,
and/or wherein the release of an active drug and/or an active reporter in presence of ROS, such as H₂O₂, is slower when the linker is (a) large and/or sterically demanding compared to the release of an active drug and/or an active reporter in presence of ROS, such as H₂O₂, when the linker is (b) small and/or sterically less demanding.

9. The compound according to any one of claims 1 to 8, wherein the sensitivity of the compound to ROS, such as to H₂O₂, is dependent on **R²,** such as is dependent on steric and/or electronic characteristics of**R²,**
and wherein the release of an active drug and/or an active reporter in presence of ROS, such as H₂O₂, is faster when **R²** is (i) small and/or sterically less demanding compared to the release of an active drug and/or an active reporter in presence of ROS, such as H₂O₂, when **R²** is (ii) large and/or sterically demanding,
and/or wherein the release of an active drug and/or an active reporter in presence of ROS, such as H₂O₂, is slower when **R²** is (ii) large and/or sterically demanding compared to the release of an active drug and/or an active reporter in presence of ROS, such as H₂O₂, when **R²** is (i) small and/or sterically less demanding,
optionally wherein **R²** is (i) small and/or sterically less demanding if **R²** is H, OH, O**R¹⁵**, N(**R¹⁵**)₂, NO₂, SO₂, P**(R¹⁶)**₃₋₅, halogen, C₁-C₅ alkyl, C₁-C₅ alkoxycarbonyl, alkyne, or alkene,
**R¹⁵** is H, OH, halogen, and
**R¹⁶** is O,
further optionally wherein **R²** is (ii) large and/or sterically demanding if **R²** is O**R¹⁵**, N(**R¹⁵**)₂, P**(R¹⁶)**₃₋₅, C₆-C₂₀ alkoxycarbonyl, aryl, or heteroaryl, wherein said aryl or said heteroaryl is optionally substituted with one or more of **R¹⁸,** preferably one or two of **R¹⁸,**
**R¹⁵** is C₁-C₁₀ alkyl, aryl, or heteroaryl,
**R¹⁶** is O**R¹⁷**,
**R¹⁷** is P, C₁-C₁₀ alkyl, C₁-C₁₀ aryl, C₁-C₁₀ heteroaryl, or ribose, and
**R¹⁸** is, at each occurrence, independently selected from H or C₁-C₅ alkyl,
such as wherein **R²** is (ii) large and/or sterically demanding if **R²** is phenyl, 2-methylphenyl, 2,6-dimethylphenyl, or 2,6-diisopropylphenyl.

10. The compound according to any one of claims 1 to 9, wherein **R⁵** is 6-Maleimidohexanamide.

11. A kit of pharmaceutical ingredients comprising
(1) a peptide or a protein which increases the level of reactive oxygen species (ROS), for example an antibody or an enzyme, preferably an L-amino acid oxidase, such as Aplysia punctata ink toxin (APIT), which comprises or consists of an amino acid sequence which is at least 90% identical, preferably at least 95% identical, more preferably at least 98% identical, even more preferably at least 99% identical, optionally identical, to the amino acid sequence of SEQ ID NO. 1, and
(2) a compound according to any one of claims 1 to 10.

12. Use of a compound according to any one of claims 1 to 10, or a kit according to claim 11, as a drug delivery system (DDS), in particular a DDS that releases an (active) drug and/or an (active) reporter in presence of reactive oxygen species (ROS), such as hydrogen peroxide (H₂O₂).

13. The compound according to any one of claims 1 to 10, or the kit according to claim 11, for use in medicine.

14. The compound according to any one of claims 1 to 10, or the kit according to claim 11, for use in a method of diagnosis, prevention and/or treatment of a disease, such as a proliferative disease, a neurodegenerative disease, a viral infection, and/or a non-viral infection in a subject in need thereof;
wherein, preferably, the proliferative disease is cancer, more preferably a cancer with solid tumor(s);
and/or wherein, preferably, the viral infection is an infection with human immunodeficiency virus (HIV), hepatitis B virus (HBV) and/or hepatitis C virus (HCV);
and/or wherein, preferably, the non-viral infection is a microbial infection.

15. A method for the diagnosis, prevention and/or treatment of a disease, such as a proliferative disease, a neurodegenerative disease, a viral infection, and/or a non-viral infection, comprising the administration of a therapeutically effective amount of the compound according to any one of claims 1 to 10, or the kit according to claim 11, to a subject in need thereof;
wherein, preferably, the proliferative disease is cancer, more preferably a cancer with solid tumor(s);
and/or wherein, preferably, the viral infection is an infection with human immunodeficiency virus (HIV), hepatitis B virus (HBV) and/or hepatitis C virus (HCV);
and/or wherein, preferably, the non-viral infection is a microbial infection;
wherein, optionally, the compound or the kit is administered via oral, intravenous and/or intratumoral administration.
